# EUROPÄISCHE PATENTSCHRIFT

(11) **EP 0 913 376 B1**
(45) Veröffentlichungstag und Bekanntmachung des Hinweises auf die Patenterteilung: **13.02.2002**
(21) Anmeldenummer: 98119890.6
(22) Anmeldetag: 21.10.1998
(51) Int. Cl.: C07B 63/02, C07C 45/85, C07C 45/00, C07C 49/403

(54) **Verfahren zur Herstellung von Cyclohexanonen**
Process for the preparation of cyclohexanones
Procédé pour la préparation de cyclohexanones

(30) Priorität: 03.11.1997 DE 19748441
(43) Veröffentlichungstag der Anmeldung: 06.05.1999
(73) Patentinhaber: MERCK PATENT GmbH, 64293 Darmstadt (DE)
(72) Erfinder: Oster, Bernd, Dr., 69493 Hirschberg (DE); Mackert, Peter, 64289 Darmstadt (DE); Pauluth, Detlef, Dr., 64372 Ober-Ramstadt (DE); Wydra, Markus, 63322 Rödermark (DE)

(56) Entgegenhaltungen:
- EP-A- 0 555 561
- GB-A- 394 979
- US-A- 3 076 810
- US-A- 3 933 916

## Beschreibung

Die Erfindung betrifft ein Verfahren zur Herstellung von Cyclohexanonen aus den entsprechenden Phenolen durch partielle Hydrierung, wobei das durch die Hydrierung erhaltene Reaktionsgemisch vor der Isolierung des Cyclohexanons Chlorsulfonsäure oder Chlorsulfonsäure-Ether-Addukten, Amidosulfonsäure, Schwefeltrioxid in unverdünnter oder mit Stickstoff oder Luft verdünnter Form oder Schwefeltrioxid-Ether-Addukten in Gegenwart von Stickstoffbasen behandelt wird.

Cyclohexanone sind bedeutende Zwischen- oder Endprodukte in der industriellen Organischen Chemie. Entsprechend substituierte Derivate stellen insbesondere wertvolle Zwischenprodukte zur Synthese von hochveredelten Endprodukten dar oder sind selbst solche Endprodukte für die Elektronik-Industrie, wie z.B. Flüssigkristalle, für den Pflanzenschutz, wie z.B. Fungizide, Insektizide, Herbizide oder Pestizide oder zur Herstellung von pharmazeutisch hochwirksamen Substanzen. Weiterhin finden Cyclohexanone in der industriellen Synthese von Kunststoffen wie Polyamiden oder im Bereich der Lack- und Farbstoffherstellung Verwendung. Eine Produktion der entsprechenden Cyclohexanone im großtechnischen Maßstab macht die möglichst wirtschaftliche und umweltverträgliche Darstellung erforderlich.

Es ist bekannt, daß Cyclohexanone durch partielle Hydrierung entsprechender Phenole hergestellt werden können. U.S. 2,829,166 und U.S. 3,076,810 lehren die Hydrierung von Phenol zu Cyclohexanon in Gegenwart eines Palladiumkatalysators. In Bull. Chem. Soc. Jpn., 65, 824 - 830 (1992) und Bull. Chem. Soc. Jpn., 65, 2955 - 2959 (1992) wird die Reaktion von Phenolen zu Cyclohexanonen in Abhängigkeit des Lösungsmittels und der Art des Katalysators beschrieben. U.S. 4,537,704 und U.S. 4,614,831 offenbaren die Herstellung von Cyclohexanoncarbonsäureestem. JP 03181438 lehrt die Herstellung von 4-lsopropylcyclohexanon durch partielle Hydrierung in Gegenwart aliphatischer und aromatischer Kohlenwasserstoffe. In JP 03109346 wird schließlich die Herstellung von Cyclohexan-1,4-dion durch partielle Hydrierung von Hydrochinon in Mesitylen oder Methanol beschrieben.

U.S. 3,933,916 beschreibt ein Verfahren zur Aufreinigung von Cyclohexanonen, die beispielsweise durch Hydrierung von Phenolen oder durch Oxidation erhalten wurden. Die auf diese Weise erhaltenen Cyclohexanone enthalten nicht näher definierte Bestandteile als Verunreinigungen, die durch Zugabe einer als Säure wirkenden Substanz durch anschließende Destillation rein erhalten werden. Einen Hinweis auf die Verwendung der speziellen Sulfiermittel der vorliegenden Anmeldung enthält U.S. 3,933,916 jedoch nicht.

In der Regel entstehen jedoch bei den partiellen Hydrierungen von Phenolen zu Cyclohexanonen Substanzgemische, die neben den gewünschten Cyclohexanonen auch die entsprechenden Cyclohexanole und die unumgesetzten Phenole enthalten. Der Aufwand zur Isolation des Cyclohexanons aus dem Reaktionsgemisch ist daher für die ökonomische Verwertung des Verfahren entscheidend.

Als industiell anwendbare Methoden zur Isolation des Cyclohexanons aus einem Reaktionsgemisch, das durch die Hydrierung eines Phenols erhalten wird, kommen in erster Linie die Destillation und/oder die Kristallisation in Frage.

Häufig sind Unterschiede der Eigenschaften der strukturell sehr ähnlichen Gemischkomponenten jedoch nur gering, so daß eine destillative Trennung des Reaktionsgemisches mit größtem Aufwand verbunden ist und zudem Ausbeuteverluste mit sich bringt. Bei Feststoffen, Verbindungen mit sehr geringen Dampfdrucken oder bei empfindlichen Substanzen ist die Destillation generell nicht ohne Weiteres einsetzbar.

Auch die Kristallisation ist als Methode zur Gemischauftrennung nicht generell durchführbar, da die hydrierten Produkte Cyclohexanon und Cyclohexanol in der Regel eine geringere Neigung zur Kristallisation aufweisen als die eingesetzten Phenole. Häufig ist daher die industrielle Herstellung von Cyclohexanonen auf diesem Wege nicht möglich.

Die der Erfindung zugrundeliegende Aufgabe bestand darin, ein Verfahren zur Herstellung von Cyclohexanonen aufzufinden, das die genannten Nachteile nicht aufweist.

Es wurde nun überraschend gefunden, daß sich Cyclohexanone in sehr guten Ausbeuten und hohen Reinheiten erhalten lassen, indem man die entsprechenden Phenole partiell hydriert und das dadurch erhaltene Reaktionsgemisch vor der Isolierung des Cyclohexanons mit Chlorsulfonsäure oder Chlorsulfonsäure-Ether-Addukten, Amidosulfonsäure, Schwefeltrioxid in unverdünnter oder mit Stickstoff oder Luft verdünnter Form oder Schwefeltrioxid-Ether-Addukten als Sulfiermittel in Gegenwart von Stickstoffbasen behandelt.

Gegenstand der Erfindung ist somit ein Verfahren zur Herstellung von Cyclohexanonen durch partielle Hydrierung von Phenolen, insbesondere ein Verfahren zur Herstellung von Cyclohexanonen der Formel I 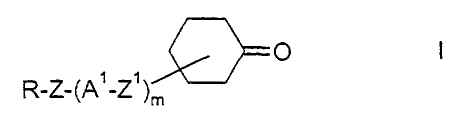 worin
- R: einen der folgenden chiralen oder achiralen Reste:
H, F, -CF₃, -OCF₃, -OCF₂CF₃ oder -OCHFCF₃, -N(R¹)₂, -COOR¹, -CON(R¹)₂, -CHO, einen unsubstituierten, einen einfach durch -CF₃ oder einen mindestens einfach durch Fluor substituierten geradkettigen oder verzweigten Alkylrest mit 1 bis 15 C-Atomen, wobei in diesen Resten auch eine oder mehrere nicht benachbarte CH₂-Gruppen jeweils unabhängig voneinander durch-S-, -O-, CO-, -CO-O-,-O-CO-, -O-CO-O- oder 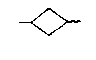 ersetzt sein können, 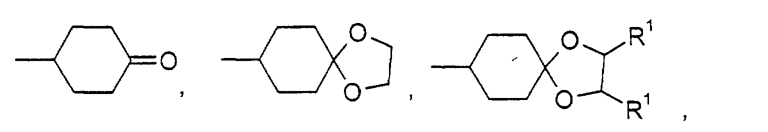 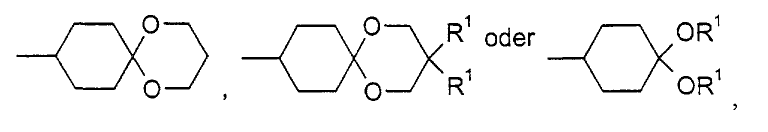
- R¹: einen Alkylrest mit 1 bis 12 C-Atomen,
- A¹: einen
(a) Cyclohexan-1,4-diylrest, worin auch eine oder mehrere nicht benachbarte CH₂-Gruppen durch -O- und/oder -S-ersetzt sein können,
(b) 1,4-Phenylenrest, worin auch eine oder zwei CH-Gruppen durch N ersetzt sein können,
(c) Rest aus der Gruppe 2,6-Dioxaboran-1,4-diyl, 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl,
wobei die Reste (a) und (b) durch ein oder mehrere Fluoratome substituiert sein können.
- Z, Z¹: unabhängig voneinander -CH₂O-, -OCH₂-, -CO-O-, -O-CO-, -CH₂S-, -SCH₂-, -CH₂CH₂- oder eine Einfachbindung
- m: 0, 1 oder 2,
bedeuten,
durch partielle Hydrierung der entsprechenden Phenole der Formel II 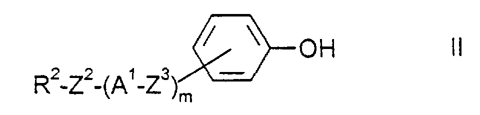 worin
- R²: die Bedeutung von R annimmt und zusätzlich auch 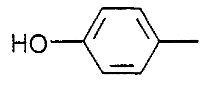 bedeutet,
- A¹, m: die für die Formel I angegebenen Bedeutungen aufweisen,
und
- Z², Z³: zusätzlich zur unter Z und Z¹ angegebenen Bedeutung auch -CH=CH- oder -C≡C-bedeutet.
dadurch gekennzeichnet, daß das durch die partielle Hydrierung erhaltene Reaktionsgemisch vor der lsolierung des Cyclohexanons mit Chlorsulfonsäure oder Chlorsulfonsäure-Ether-Addukten, Amidosulfonsäure, Schwefeltrioxid in unverdünnter oder mit Stickstoff oder Luft verdünnter Form oder Schwefeltrioxid-Ether-Addukten in Gegenwart von Stickstoffbasen behandelt wird.

Sofern A¹ in einer Verbindung der Formel I oder II mehrfach vorkommt, können sie dieselbe oder verschiedene Bedeutungen annehmen. Dasselbe gilt auch für alle anderen mehrfach auftretenden Gruppen.

Das erfindungsgemäße Verfahren eignet sich insbesondere zur Herstellung von Zwischenstufen für die Flüssigkristallsynthese.

Bevorzugt werden solche Verbindungen der Formel II für das Verfahren verwendet, in denen sich der Rest R²-Z²-(A¹-Z³)ₘ in para-Position zur phenolischen Hydroxygruppe befindet, wodurch in 4-Stellung substituierte Cyclohexanone der Formel I erhalten werden.

Der Einfachheit halber bedeuten im folgenden Phe eine 1,4-Phenylengruppe, worin auch eine oder zwei CH-Gruppen durch N ersetzt sein können, wobei eine 1,4-Phenylengruppe auch durch ein oder zwei Fluoratome substituiert sein kann, Cyc einen kans-Cydohexan-1,4-diylrest, Dio einen trans-Dioxan-1,4-diylrest. W bedeutet die folgende Gruppe:

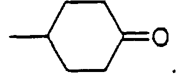

Die nach dem erfindungsgemäßen Verfahren hergestellten bevorzugten Verbindungen umfassen diejenigen der Formeln Ia bis Ir:

| | |
|---|---|
| W-Z-W | la |
| W-A¹-Z¹-W | Ib |
| R-A¹-Z¹-W | Ic |
| R-A¹-Z¹-A¹-Z¹-W | Id |
| W-OOC-W | le |
| W-CH₂CH₂-W | If |
| W-Phe-W | Ig |
| W-Cyc-W | lh |
| W-Dio-W | li |
| W-Phe-OOC-W | lj |
| W-Cyc-CH₂CH₂-W | lk |
| R-Phe-W | II |
| R-Cyc-W | Im |
| R-Dio-W | In |
| R-Phe-OOC-W | lo |
| R-Cyc-CH₂CH₂-W | lp |
| R-Cyc-Phe-W | Iq |
| R-Z-W | Ir |

In den bevorzugten Verbindungen der vor- und nachstehenden Formeln bedeutet R bevorzugt eine Alkyl- oder Alkoxygruppe mit 1 bis 10 C-Atomen, H, F, -CF₃, -OCF₃, -OCF₂CF₃, -N(R¹)₂, -COOR¹, -CON(R¹)₂, -CHO, 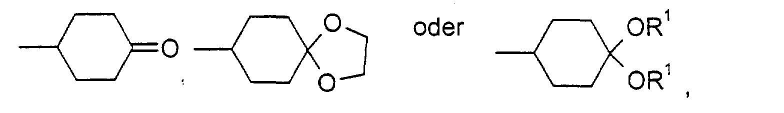 insbesondere eine geradkettige Alkyl- oder Alkoxygruppe mit 1 bis 7 C-Atomen, H, F, -OCF₃, -COOR¹, -CCN(R¹)₂,

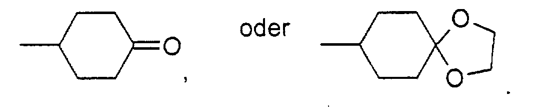

Insbesondere finden solche Verbindungen der Formel II für das erfindungsgemäße Verfahren Verwendung, worin m 0 oder 1 bedeutet. Eine besonders bevorzugte Verbindung, die als Ausgangsmaterial für das erfindungsgemäße Verfahren eingesetzt wird, ist das 4,4'-Dihydroxybiphenyl. Hierdurch wird 4,4'-Dioxobicyclohexyl erhalten. Ein weitere bevorzugte Ausgangsverbindung ist das 4,4'-Dihydroxystilben, aus dem nach dem erfindungsgemäßen Verfahren 4-(4'-Oxocyclohexylethyl)cyclohexanon erhalten wird.

A¹ ist bevorzugt Cyc, oder Phe. In den Verbindungen der vor- und nachstehenden Formeln bedeutet Phe vorzugsweise eine 1,4-Phenylengruppe (Ph), eine ein- oder zweifach durch F substituierte 1,4-Phenylengruppe (PheF) eine Pyrimidin-2,5-diyl- (Pyr), eine Pyridin-2,5-diyl- (Pyn), eine Pyrazin-3,6-diyl- oder eine Pyridazin-2,5-diyl-Gruppe, insbesondere bevorzugt Ph, PheF, Pyr oder Pyn. Vorzugsweise enthalten die nach dem erfindungsgemäßen Verfahren hergestellten Verbindungen nicht mehr als eine 1,4-Phenylengruppe, worin eine oder zwei CH-Gruppen durch N ersetzt sind. Cyc bedeutet vorzugsweise eine Cyclohexan-1,4-diylgruppe. Insbesondere bevorzugt sind auch Verbindungen der Formel I, worin zumindest eine der Gruppen A¹ eine in 1- oder 4-Position durch F substituierte Cyclohexan-1,4-diylgruppe bedeutet und das Fluoratom sich in axialer Position befindet, d.h. die Gruppe A¹ die folgende Struktur aufweist:

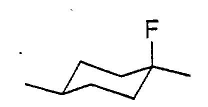

Weiterhin bevorzugt sind Verbindungen der Formel I und der nachstehenden Teilformeln, die eine 2,3-Difluor-, 2,6-Difluor- oder 3,5-Difluor-1,4-phenylengruppe enthalten.

Die Gruppen Z und Z¹ bedeuten bevorzugt eine Einfachbindung, -COO-, -OOC- oder eine -CH₂CH₂-Gruppe, insbesondere eine Einfachbindung oder eine-CH₂CH₂-Gruppe. Verbindungen der Formel I, in denen nicht mehr als eine der Gruppen -CH₂CH₂-, -COO- oder -OOC-vorkommt, sind bevorzugt.

R¹ ist bevorzugt eine geradkettige Alkylgruppe mit 1 bis 5 C-Atomen, insbesondere Methyl, Ethyl, n-Propyl oder n-Butyl.

Falls R eine Alkylgruppe bedeutet, in der auch eine CH₂-Gruppe (Alkoxy bzw. Oxaalkyl) durch ein O-Atom ersetzt sein kann, so kann sie geradkettig oder verzweigt sein. Vorzugsweise hat sie 2, 3, 4, 5, 6, 7, 8, 9 oder 12 C-Atome und bedeutet demnach bevorzugt Ethyl, Propyl, Butyl, Pentyl, Hexyl, Heptyl, Octyl, Nonyl, Decyl, Ethoxy, Propoxy, Butoxy, Pentoxy, Hexoxy, Heptoxy, Octoxy, Nonoxy oder Decoxy, ferner auch Undecyl, Dodecyl, Undecoxy, Dodecoxy, 2-Oxapropyl (= 2-Methoxymethyl), 2-Oxabutyl(= Methoxyethyl) oder 3-Oxabutyl (= 2-Ethoxymethyl), 2-, 3- oder 4-Oxapentyl, 2-, 3-, 4- oder 5-Oxahexyl, 2-, 3-, 4-, 5- oder 6-Oxaheptyl. Insbesondere bevorzugt sind Hexyl, Pentyl, Butyl, n-Butyl, Propyl, i-Propyl, Methyl und Ethyl, insbesondere Propyl und Pentyl; besonders bevorzugte Alkoxygruppen sind Hexoxy, Pentoxy, n-Butoxy, Propoxy, i-Propoxy, Methoxy und Ethoxy, insbesondere Ethoxy und n-Butoxy. Verbindungen der vor- und nachstehenden Formeln mit verzweigten Flügelgruppen R können von Bedeutung sein. Verzweigte Gruppen dieser Art enthalten in der Regel nicht mehr als zwei Kettenverzweigungen. R ist vorzugsweise eine geradkettige Gruppe oder eine verzweigte Gruppe mit nicht mehr als einer Kettenverzweigung.

Bevorzugte verzweigte Reste sind Isopropyl, 2-Butyl (= 1-Methylpropyl), Isobutyl (= 2-Methylpropyl), tert.-Butyl, 2-Methylbutyl, Isopentyl (= 3-Methylbutyl), 2-Methylpentyl, 3-Methylpentyl, 4-Methylpentyl, 2-Ethylhexyl, 5-Methylhexyl, 2-Propylpentyl, 6-Methylheptyl, 7-Methyloctyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl. Der Rest R kann auch ein optisch aktiver organischer Rest mit einem asymmetrischen Kohlenstoffatom sein. Vorzugsweise ist dann das asymmetrische Kohlenstoffatom mit zwei unterschiedlich substituierten C-Atomen, einem H-Atom und einem Substituenten ausgewählt aus der Gruppe Methyl oder Methoxy verknüpft. Der optisch aktive organische Rest R hat vorzugsweise die Formel 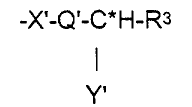 worin
- X': -O-, -S- oder eine Einfachbindung,
- Q': eine Alkylgruppe mit 1 bis 5 C-Atomen, worin auch eine nicht mit X' verknüpfte CH₂-Gruppe durch -O- ersetzt sein kann, oder eine Einfachbindung,
- Y': F, -CF₃, Methyl oder Methoxy und
- R³: eine von Y' verschiedene Alkylgruppe mit 1 bis 5 C-Atomen, worin auch eine oder zwei nicht benachbarte CH₂-Gruppen durch -S- oder -O- ersetzt sein können,
bedeutet.
- X': ist vorzugsweise eine Einfachbindung,
- Q': bedeutet vorzugsweise -CH₂-, -CH₂CH₂-, -CH₂CH₂CH₂- oder eine Einfachbindung, insbesondere bevorzugt eine Einfachbindung,
- Y': ist vorzugsweise -CH₃ oder F, insbesondere bevorzugt F,
- R³: ist vorzugsweise geradkettiges oder verzweigtes Alkyl oder Alkoxy mit 2 bis 5, insbesondere mit 2 bis 3 C-Atomen.

Ganz besonders eignet sich das erfindungsgemäße Verfahren zur Herstellung der Cyclohexanone der Formeln I1 bis I19: 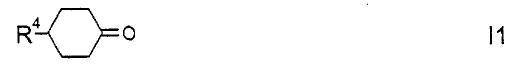 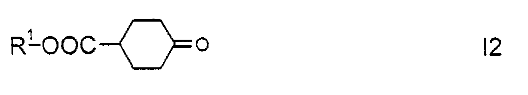 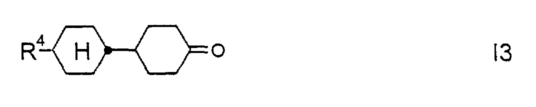 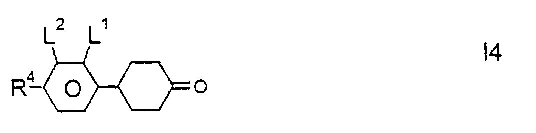 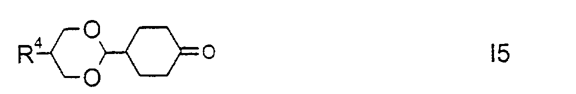 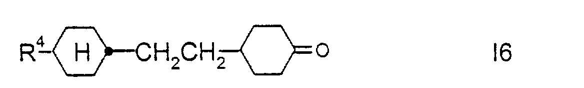 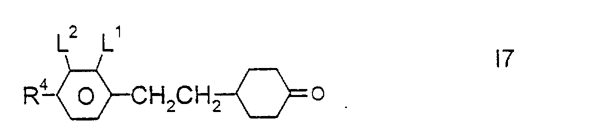 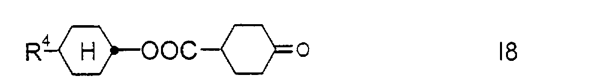 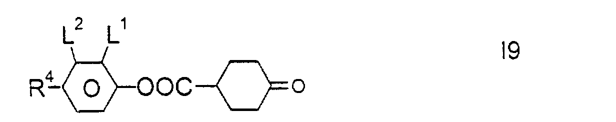 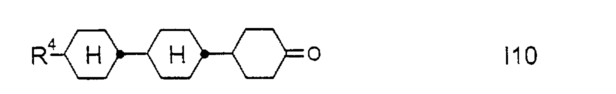 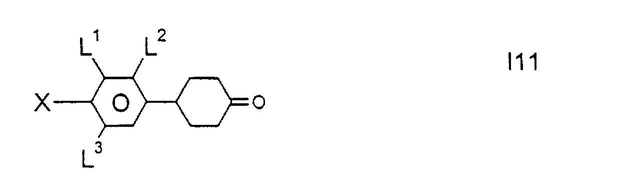 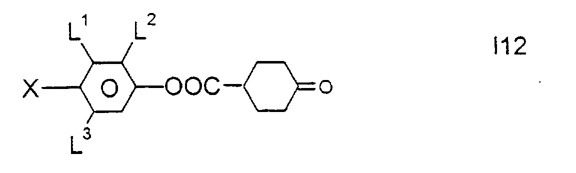 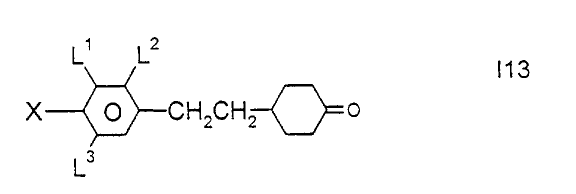 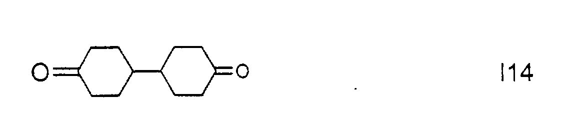 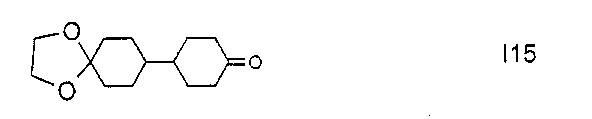 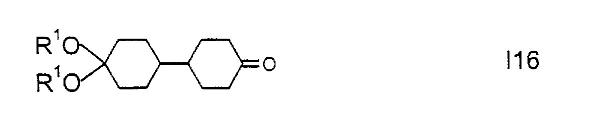 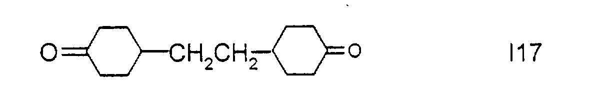 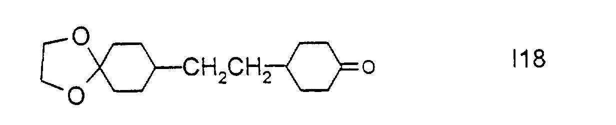 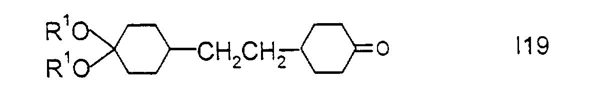 wobei
- R¹: die oben angegebene Bedeutung aufweist,
- R⁴: -N(R¹)₂, -CON(R¹)₂, einen geradkettigen Alkylrest mit 1 bis 12 C-Atomen, wobei in diesem Rest auch eine oder mehrere CH₂-Gruppen durch-O- oder -CO- so ersetzt sein können, daß O-Atome nicht direkt miteinander verknüpft sind, bedeutet,
- L¹, L², L³: unabhängig voneinander H oder F
und
- X: F, -CF₃, -OCF₃, -OCF₂CF₃ oder -OCHFCF₃
bedeutet.

Ganz besonders bevorzugte Cyclohexanone, die sich nach dem erfindungsgemäßen Verfahren herstellen lassen, sind die Verbindungen der Formeln I1, I2, I3, I4, I5, I7, I9, I13, I14, I15, I17und I18.

Die als Ausgangsstoffe benötigten Phenole sind entweder bekannt oder werden nach an sich bekannten Methoden hergestellt, wie sie in der Literatur beschrieben sind (z.B. Standardwerken wie Houben-Weyl, Methoden der organischen Chemie, Georg-Thieme-Verlag, Stuttgart oder Organikum, 17. Auflage, VEB Deutscher Verlag der Wissenschaften, Berlin 1988) und zwar unter Reaktionsbedingungen, die für die genannten Umsetzungen bekannt und geeignet sind. So sind die entsprechenden Phenole z.B. durch Alkalischmelzen aromatischer Sulfonsäuren, durch Verkochen von Diazoniumsalzlösungen oder durch Oxidation von Phenylmetallderivaten herstellbar. Man kann aber auch von an sich bekannten, hier nicht näher erwähnten Varianten Gebrauch machen. Bevorzugte Phenole, die als Ausgangsverbindungen für das erfindungsgemäße Verfahren dienen, können z.B. nach den folgenden Schemata erhalten werden:

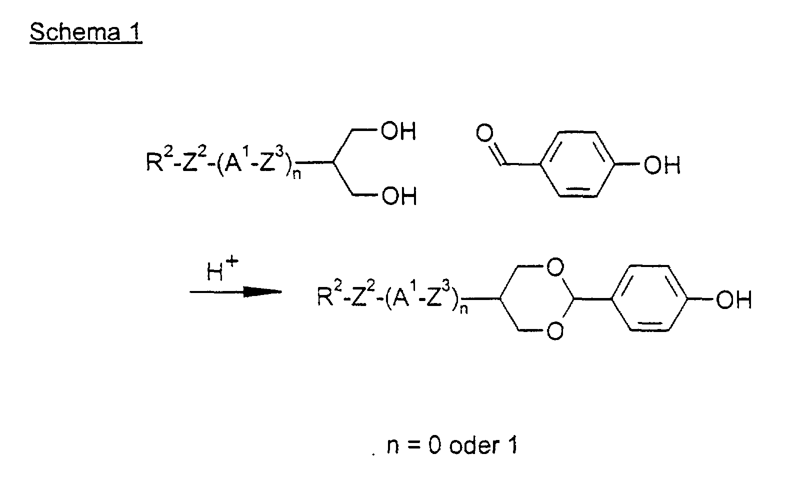

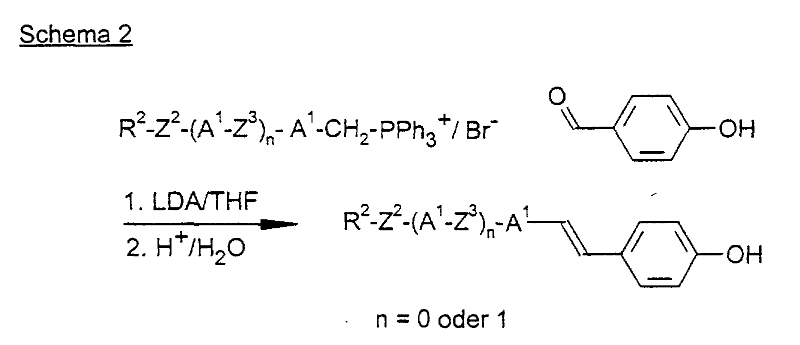

Die Reaktionsdurchführung ist einfach, wobei zunächst das betreffende Phenol in gewohnter Weise bei Temperaturen von -20 bis +250°C, vorzugsweise bei -20 bis +200°C, ganz besonders bevorzugt bei +20 bis +200°C und bei normalem oder erhöhtem Wasserstoffdruck, vorzugsweise bei erhöhtem Wasserstoffdruck, partiell hydriert wird. Die Hydrierungen werden bevorzugt bei einem Druck von 1-50 bar, vorzugsweise bei 2 bis 10 bar durchgeführt. Die Dauer der Hydrierung hängt von den gewählten Reaktionsbedingungen ab.

Die Art des bei der Hydrierung einzusetzenden Katalysators ist an sich unkritisch. Üblicherweise werden die in der organischen Chemie gebräuchlichen Katalysatoren verwendet (z.B. Organikum, 15. Aufl., VEB, 1976, S. 359 ff oder Houben-Weyl, Methoden der organischen Chemie, Georg-Thieme-Verlag, Stuttgart).
Es können Katalysatoren mit reduzierter Hydrieraktivität verwendet werden. Edelmetallkatalysatoren, die Platin, Rhodium oder Iridium in metallischer oder oxidischer Form enthalten oder ein Palladiumkatalysator sind bevorzugt. Die Katalysatoren können in homogener Weise verwendet werden oder immobilisiert auf einen Träger wie z.B. Aktivkohle, Aluminiumoxid, Siliziumoxid, Calciumcarbonat und Bariumsulfat zum Einsatz kommen. Palladium-Katalysatoren, die auf Aktivkohle aufgebracht sind (Pd/C), sind besonders bevorzugt.
Ein besonders geeigneter Hydrierkatalysator enthält metallisches Palladium auf einem geeigneten Trägerstoff, bevorzugt Aktivkohle, das mit Alkali- oder Erdalkalicarbonat oder -hydroxid dotiert wurde. Der Anteil der Alkali oder Erdalkali-Dotierung an der Gesamtmasse des Katalysators macht in der Regel 0.5 bis 25 Gew.-%, insbesondere 1 bis 20 Gew.-% aus. Bevorzugt wird mit Alkalicarbonat dotiert, insbesondere mit Natrium oder Kaliumcarbonat. Die Dotierung kann z.B. in der Weise erfolgen, daß das Palladium oder die Palladium/Träger-Mischung mit Alkali- oder Erdalkalicarbonat in wässriger Lösung behandelt und anschließend das Wasser im Vakuum entfernt wird.

In der Regel benötigt man auf 1 Teil des zu hydrierenden Phenols 0.001 bis 0.5 Teile, vorzugsweise 0.01 bis 0.25 Teile des Katalysators, wobei in der Regel die aktive Komponente des Katalysators, d.h. das Übergangsmetall, etwa 1 bis 30, vorzugsweise 2 bis 15 Gew.-% des Gesamtkatalysators ausmacht.

Durch die Hydrierung unter den genannten Bedingungen werden im allgemeinen nur Phenole oder isolierte Dreifach- oder Doppelbindungen hydriert. Benzolringe, die keine OH-Gruppe tragen, werden in der Regel nicht angegriffen.

Sowohl die Hydrierung als auch die sich anschließende Sulfierung kann in der Schmelze oder in Lösungsmitteln durchgeführt werden. Vorzugsweise wird in Gegenwart von organischen Lösungsmitteln gearbeitet. Im allgemeinen wird im erfindungsgemäßen Verfahren für die Hydrierung und die Sulfierung dasselbe Lösungsmittel verwendet.

Geeignete Lösungsmittel sind insbesondere aromatische Lösungsmittel, wie z.B. Benzol, Toluol, Xylole, Mesitylen, Anisol, Phenetol oder Tetrahydronaphthalin. Weiterhin können auch gesättigte Kohlenwasserstoffe, wie Cyclohexan, n-Hexan oder n-Octan, Ester wie Methylacetat, Ethylacetat, Propylacetat oder Butylacetat oder Ether wie Diethylether, Methyl-tert-butylether, Tetrahydrofuran oder Dioxan verwendet werden. Alkohole,wie z.B. Methanol, Ethanol, n-Propanol, i-Propanol, n-Butanol, i-Butanol oder tert-Butanol eignen sich für das erfindungsgemäße Verfahren nur dann, wenn sie nach der Hydrierung und vor der Zugabe des Sulfiermittels entfernt und/oder durch die genannten nichtalkoholischen Lösungsmittel oder halogenierte Kohlenwasserstoffe wie z.B. Dichlormethan, Trichlormethan, Dichlorethylen oder Trichlorethylen ersetzt werden. Bevorzugte Lösungsmittel für das erfindungsgemäße Verfahren sind Benzol, Toluol, Xylole, Mesitylen, Anisol, Phenetol oder Tetrahydronaphthalin, insbesondere Toluol, o-Xylol oder Mesitylen. Mischungen der genannten Lösungsmittel können ebenfalls Verwendung finden.

Die Menge des Lösungsmittels ist nicht kritisch, im allgemeinen können 10 bis 10000 g Lösungsmittel je Mol des zu hydrierenden Phenols zugesetzt werden.
Cosolventien, wie Tetramethylethylendiamin (TMEDA) oder Kronenether, wie 18-Crown-6, können diesen Lösungsmitteln zugesetzt werden. Zusatz von substituierten Pyridinen wie z.B. 3- oder 4-Dimethylaminopyridin ist ebenfalls möglich.
Generell sollten möglichst wasserfreie Lösungsmittel zum Einsatz kommen. Während für den Hydrierungsschritt des erfindungsgemäßen Verfahrens eventuell vorhandenes Wasser nicht stört, sollte gegenwärtiges Wasser z.B. durch Destillation entfernt werden, bevor die Sulfierung stattfindet. Nur bei Anwendung entsprechend größerer Mengen des Sulfiermittels kann in der Reaktionsmischung vorhandenes Wasser vernachlässigt werden.

Die durch die Hydrierung des betreffenden Phenols erhaltene Reaktionsmischung, die je nach Hydrierbedingungen und eingesetzter Ausgangsverbindung unterschiedliche Anteile des Cyclohexanons, des entsprechenden Cyclohexanols und/oder des unumgesetzten Phenols enthält, wird vorzugsweise mit einer Menge an Sulfiermitteln behandelt, die den molaren Anteilen des Cyclohexanols und Phenols entspricht. Ebenfalls ist es möglich, einen Überschuß an Sulfiermitteln einzusetzen. Bei der Behandlung des durch die partielle Hydrierung erhaltenen Reaktionsgemisches mit Sulfiermitteln wird die Hydroxy-Gruppe des unerwünschten Cyclohexanols in das korrespondierenden Sulfat oder in den entsprechenden Schwefelsäurehalbester überführt. Phenole können je nach verwendetem Sulfiermittel in die jeweiligen Sulfate bzw. Schwefelsäurehalbester aber auch in die entsprechenden Phenolsulfonsäuren überführt werden.

Es ist ein besonderes Merkmal des erfindungsgemäßen Verfahrens, daß sich die Sulfiermittel unter den gewählten Bedingungen ausschließlich mit den im durch die partielle Hydrierung erhaltenen Reaktionsgemisch enthaltenen Komponenten Cyclohexanol und/oder Phenol umsetzen. Eine ausbeuteeinschränkende Reaktion des Cyclohexanons zu den entsprechenden α-Ketosulfonsäuren, wie sie für eine Reihe von Ketonen bekannt ist (z.B. Houben-Weyl, Methoden der Organischen Chemie, Bd. 9, S. 362 f, 4. Aufl., Georg-Thieme-Verlag, Stuttgart 1955), findet nicht statt.

Bevorzugt werden Chlorsulfonsäure, Schwefeltrioxid in unverdünnter oder mit Stickstoff oder Luft verdünnter Form oder Schwefeltrioxid-Stickstoffbase-Addukte eingesetzt. Insbesondere bevorzugt sind Schwefeltrioxid-Stickstoffbase-Addukte. Diese Schwefeltrioxid-Stickstoffbase-Addukte sind kommerziell erhältlich oder durch einfaches Zusammengeben der Komponenten darstellbar.

Die Wahl der Stickstoffbase für das erfindungsgemäße Verfahren ist unkritisch. Geeignete Stickstoffbasen sind beispielsweise Stickstoffheterocyclen, Amine oder Amidine. Vorzugsweise werden solche Stickstoffbasen eingesetzt, worin keine H-Atome direkt an ein N-Atom gebunden sind. Bevorzugte Stickstoffbasen sind Pyridine, Pyrimidine, Pyridazine, Trialkylamine oder Dialkylarylamine, wobei die Alkylreste in den Trialkylaminen und Dialkylarylaminen identisch oder unterschiedlich sein können. Insbesondere bevorzugt sind Imidazol, Pyridin, p-Dimethylaminopyridin, m-Dimethylaminopyridin, o-Dimethylaminopyridin, Pyrimidin, Trimethylamin, Triethylamin, Tripropylamin, Triisopropylamin, Dimethylanilin, Diethylanilin. Ganz besonders bevorzugt sind Pyridin, p-Dimethylaminopyridin, m-Dimethylaminopyridin, Trimethylamin, Triethylamin und Dimethylanilin. Es können auch Gemische der genannten Stickstoffbasen Verwendung finden.

Das erfindungsgemäße Verfahren wird vorteilhaft in der Weise durchgeführt, daß das Sulfiermittel bei Temperaturen von -20°C bis zum Siedepunkt der jeweils geringst flüchtigen Komponente des Reaktionsgemisches, insbesondere zwischen 0°C und 80°C, unter Rühren zum durch die Hydrierung erhaltenen Reaktionsgemisch gegeben wird. Die Dauer der Sulfierung beträgt im allgemeinen 0.1 bis 10 Stunden, bevorzugt 0.2 bis 2 Stunden.

Das Verfahren kann auch in der Weise erfolgen, daß das Reaktionsgemisch, das durch die Hydrierung erhalten wurde, unter Rühren zum Sulfiermittel oder zu einer Lösung des Sulfiermittels gegeben wird. Die Isolation des Cyclohexanons kann im Anschluß an die Sulfierung in gewohnter Weise erfolgen. Beispielsweise kann die Stickstoff base im Anschluß an die Sulfierung zugegeben werden, so daß die gebildeten Sulfate und/oder Sulfonate durch eine Filtration entfernt werden können. Die Filtration erfolgt bevorzugt durch eine Lage von Adsorbentien. Als Adsorbentien dienen im erfindungsgemäßen Verfahren alle bekannten polaren Adsorbtionsmittel, die geeignet sind, die entsprechenden Sulfate bzw. Sulfonate der Cyclohexanole und/oder Phenole chemisch oder physikalisch ausreichend stark zu binden. Beispiele hierfür sind Kieselgel, Aluminiumoxid in neutraler, saurer oder basischer Form aber auch andere gebräuchliche Medien, wie z.B. Molekularsieb oder Mischungen von Adsorbentien der genannten Art.

Durch die Filtration wird direkt oder gegebenenfalls nach Entfernung des Lösungsmittels im Vakuum, das gewünschte Cyclohexanon erhalten.

Ebenso ist es möglich, in Fällen in denen das gewünschte Cyclohexanon einen relativ hohen Dampfdruck aufweist, die mit Sulfiermitteln und Stickstoffbasen behandelte Reaktionsmischung direkt einer Destillation zu unterziehen.

Das erfindungsgemäße Verfahren kann auch in der Weise durchgeführt werden, daß nach der Sulfierung des Reaktionsgemisches, das durch die Hydrierung erhalten wurde, mit Wasser oder einer wässrigen Lösung einer Base, z.B. Alkali- oder Erdalkalihydroxid, Alkali- oder Erdalkalicarbonat oder Alkali- oder Erdalkalihydrogencarbonat, bevorzugt Alkali- oder Erdalkalihydroxid, versetzt wird und die Sulfate und/oder Sulfonate der Cyclohexanole und/oder Phenole durch Auswaschen entfernt werden. Nach Trocknung der organischen Phase wird direkt oder gegebenenfalls nach Entfernung des Lösungsmittels das Cyclohexanon erhalten.
In einer besonderen Ausführungsform des erfindungsgemäßen Verfahrens wird ein Schwefeltrioxid-Stickstoffbase-Addukt, insbesondere das Schwefeltrioxid-Pyridin-Addukt, als Feststoff oder in Lösung unter Rühren zum durch die partielle Hydrierung erhaltenen Reaktionsgemisch gegeben. Das Verfahren kann bevorzugt auch in der Weise durchgeführt werden, daß das Reaktionsgemisch, das durch die partielle Hydrierung erhalten wurde, zu einer Lösung von Stickstoffbase und Schwefeltrioxid gegeben wird. In einer weiteren bevorzugten Ausführungsform ist es auch möglich, Schwefeltrioxid unverdünnt oder verdünnt unter Kühlung in eine Lösung des Cyclohexanons, Cyclohexanols und/oder Phenols und der Stickstoffbase einzuleiten.
Zur Isolation des Cyclohexanons werden in diesen bevorzugten Ausführungsformen die gebildeten Sulfate und/oder Sulfonate durch eine Filtration entfernt und der Rückstand gegebenenfalls vom Lösungsmittel befreit. Die Filtration erfolgt bevorzugt durch eine Lage der obengenannten Adsorbentien.

Es kann vorteilhaft sein, nach der Entfernung der Sulfate und/oder Sulfonate der Cyclohexanole und/oder Phenole durch Filtration und der Entfernung des Lösungsmittels, eine Destillation oder Kristallisation anzuschließen.

Die nach dem erfindungsgemäßen Verfahren hergestellten Cyclohexanone der Formel I aus den Phenolen der Formel II stellen bedeutende Zwischen- bzw. Endprodukte in der organischen industriellen Chemie dar. Entsprechend substituierte Derivate stellen insbesondere wertvolle Zwischenprodukte zur Synthese hochveredelter Endprodukte dar, bzw. sind selbst solche Endprodukte für die Elektronikindustrie, wie z.B. Flüssigkristalle, für den Pflanzenschutz, wie z.B. Fungizide, Insektizide, Herbizide oder Pestizide oder zur Herstellung von pharmazeutisch hochwirksamen Substanzen.

Auch ohne weitere Ausführungsformen wird davon ausgegangen, daß ein Fachmann die obige Beschreibung im weitesten Umfang nutzen kann. Die bevorzugten Ausführungsformen sind deswegen lediglich als beschreibende, keineswegs als in irgendeiner Weise limitierende Offenbarung aufzufassen.

Die folgenden Beispiele sollen die Erfindung erläutern, ohne sie zu begrenzen. Sofern nichts anderes angegeben ist, bedeuten Prozentangaben Gewichtsprozent. Alle Temperaturen sind in Grad Celsius angegeben.

### Beispiele:

### Herstellung des Hydrierkatalysators

Zu einer Suspension von 20 g Palladium auf Aktivkohle (5 Gew-%) in 100 ml entionisiertem Wasser fügt man unter Rühren eine Lösung von 3 g Natriumcarbonat in 100 ml Wasser zu. Man läßt 0.5 - 2 Stunden weiterrühren und entfernt anschließend den Großteil des Wassers durch Vakuumdestillation am Rotationsverdampfer bei 50°C. Es resultieren Natriumcarbonat-dotierte Katalysatorpasten mit einer Wasserrestfeuchte von 20 - 40 % (w/w).

### Beispiel 1

### trans-4-(4-n-Propylcyclohexyl)cyclohexanon:

Eine Lösung von 500 g 4-(4-n-Propylcyclohexyl)phenol in 5 I Toluol wird bei 5 bar Wasserstoffdruck und 120°C in Gegenwart von 50 g Palladium auf Aktivkohle (5 Gew-%) hydriert. Das Reaktionsgemisch wird filtriert und durch azeotrope Destillation vom Wasser befreit. Anschließend wird mit 110 g Pyridinsulfon (Pyridin-Schwefeltrioxid-Kompiex) versetzt, 1 Stunde bei 50°C gerührt und mit basischem Aluminiumoxid filtriert. Nach Entfernen des Lösungsmittels verbleiben 433 g (86 % Ausbeute) des trans-4-(4-n-Propylcyclohexyl)cyclohexanons als farblos erstarrte Schmelze mit einer Reinheit von 99.5 % (GC).

### Beispiel 2

### Cyclohexan-4-on-carbonsäureethylester:

Eine Lösung von 50 g 4-Hydroxybenzoesäureethylester in 500 ml Toluol wird bei 2.5 bar und 110°C in Gegenwart von Palladium auf Aktivkohle hydriert. Das Reaktionsgemisch wird filtriert und durch azeotrope Destillation vom Wasser befreit. Anschließend wird mit 10 g Pyridinsulfon versetzt und 1 Stunde bei 50°C gerührt. Nach Filtration mit basischem Aluminiumoxid und Entfernen des Lösungsmittels verbleiben 49.6 g (97 % Ausbeute) des Cyclohexan-4-on-carbonsäureethylesters mit einer Reinheit von 94,1 % (GC).

### Beispiel 3

### 4,4'-Bicyclohexandion:

Eine Suspension von 50 g 4,4'-Biphenyldiol in 500 ml Toluol wird bei 5 bar und 125°C in Gegenwart von Palladium auf Aktivkohle hydriert.Das Reaktionsgemisch wird filtriert und durch azeotrope Destillation vom Wasser befreit. Anschließend wird mit 29 g Pyridinsulfon versetzt und 1 Stunde bei 50°C gerührt. Nach Filtration mit basischem Aluminiumoxid und Entfernen des Lösungsmittels verbleiben 34.4 g (66 % Ausbeute) des 4,4'-Bicyclohexandions als farblose Kristalle mit einer Reinheit von 99.0 % (GC).

### Beispiele 4 - 7

Analog zu den vorhergehenden Beispielen wurden unter Verwendung des mit Natriumcarbonat dotierten Palladium-Katalysators die folgenden Cyclohexanone erhalten:

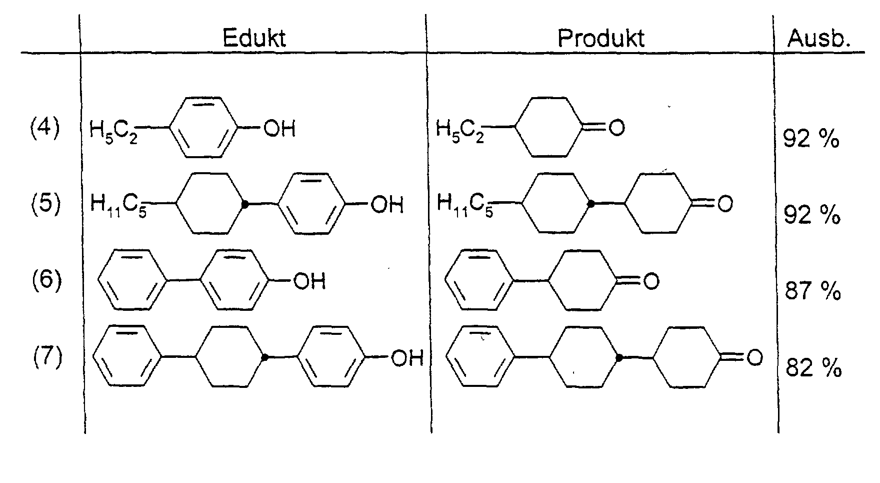

## Patentansprüche

1. Verfahren zur Herstellung von Cyclohexanonen aus den entsprechenden Phenolen durch partielle Hydrierung, **dadurch gekennzeichnet, daß** das durch die Hydrierung erhaltene Reaktionsgemisch vor der Isolierung des Cyclohexanons mit Chlorsulfonsäure oder Chlorsulfonsäure-Ether-Addukten, Amidosulfonsäure, Schwefeltrioxid in unverdünnter oder mit Stickstoff oder Luft verdünnter Form oder Schwefeltrioxid-Ether-Addukten in Gegenwart von Stickstoffbasen behandelt wird.

2. Verfahren nach Anspruch 1 zur Herstellung von Cyclohexanonen der Formel I 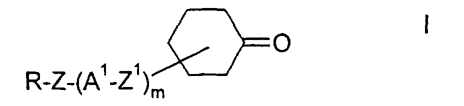 worin
R einen der folgenden chiralen oder achiralen Reste: H, F, -CF₃, -OCF₃, -OCF₂CF₃ oder -OCHFCF₃, -N(R¹)₂, -COOR¹, -CON(R¹)₂, -CHO, einen unsubstituierten, einen einfach durch -CF₃ oder einen mindestens einfach durch Fluor substituierten geradkettigen oder verzweigten Alkylrest mit 1 bis 15 C-Atomen, wobei in diesen Resten auch eine oder mehrere nicht benachbarte CH2-Gruppen jeweils unabhängig voneinander durch-S-, -O-, CO-, -CO-O-,-O-CO-, -O-CO-O- oder 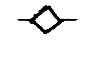 ersetzt sein können, 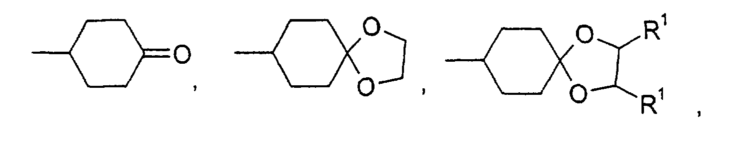 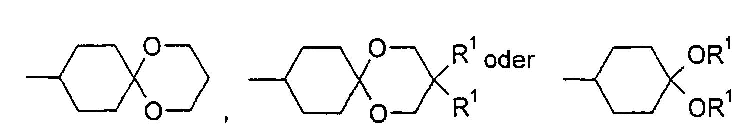
R¹ einen Alkylrest mit 1 bis 12 C-Atomen,
A1 einen
(a) Cyclohexan-1,4-diylrest, worin auch eine oder mehrere nicht benachbarte CH₂-Gruppen durch -O- und/oder -S- ersetzt sein können,
(b) 1,4-Phenylenrest, worin auch eine oder zwei CH-Gruppen durch N ersetzt sein können,
(c) Rest aus der Gruppe 2,6-Dioxaboran-1,4-diyl, 1,4-Bicyclo(2,2,2)-octylen, Piperidin-1,4-diyl, Naphthalin-2,6-diyl, Decahydronaphthalin -2,6-diyl und 1,2,3,4-Tetrahydronaphthalin-2,6-diyl,
wobei die Reste (a) und (b) durch ein oder mehrere Fluoratome substituiert sein können,
Z, Z¹ unabhängig voneinander -CH₂O-, -OCH₂-, -CO-O-, -O-CO-, -CH₂S-, -SCH₂-, -CH₂CH₂- oder eine Einfachbindung,
m 0, 1 oder 2,
bedeuten,
durch partielle Hydrierung der entsprechenden Phenole der Formel II 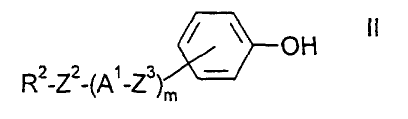 worin
R2 die Bedeutung von R annimmt und zusätzlich auch 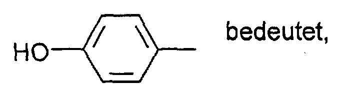
A¹, m die für die Formel I angegebenen Bedeutungen aufweisen,
und
Z², Z³ zusätzlich zur unter Z und Z¹ angegebenen Bedeutung auch - CH=CH- oder -C≡C-bedeutet.

3. Verfahren nach einem der Ansprüche 1 oder 2, **dadurch gekennzeichnet, daß** das durch die Hydrierung erhaltene Reaktionsgemisch mit einer Menge an Chlorsulfonsäure oder Chlorsulfonsäure-Ether-Addukten, Amidosulfonsäure, Schwefeltrioxid in unverdünnter oder mit Stickstoff oder Luft verdünnter Form oder Schwefeltrioxid-Ether-Addukten behandelt wird, die den molaren Anteilen des Cyclohexanols und Phenols entspricht.

4. Verfahren nach einem der Ansprüche 1,2 oder 3, **dadurch gekennzeichnet, daß** das durch die Hydrierung erhaltene Reaktionsgemisch bei Temperaturen von -20°C bis zum Siedepunkt der jeweils geringst flüchtigen Komponente des Reaktionsgemisches, mit Chlorsuifonsäure oder Chlorsulfonsäure-Ether-Addukten, Amidosulfonsäure, Schwefeltrioxid in unverdünnter oder mit Stickstoff oder Luft verdünnter Form oder Schwefeltrioxid-Ether-Addukten umgesetzt wird.

5. Verfahren nach einem der Ansprüche 1, 2, 3 oder 4, **dadurch gekennzeichnet, daß** das durch die Hydrierung erhaltene Reaktionsgemisch mit Schwefeltrioxid-Pyridin-Addukt behandelt wird.

6. Verfahren nach einem der vorhergehenden Ansprüche, **dadurch gekennzeichnet, daß** das durch die Hydrierung erhaltene und mit Chlorsulfonsäure oder Chlorsulfonsäure-Ether-Addukten, Amidosulfonsäure, Schwefeltrioxid in unverdünnter oder mit Stickstoff oder Luft verdünnter Form oder Schwefeltrioxid-Ether-Addukten in Gegenwart von Stickstoffbasen behandelte Reaktionsgemisch einer Filtration unterzogen wird.

7. Verfahren nach einem der vorhergehenden Ansprüche, **dadurch gekennzeichnet, daß** die Hydrierung der Phenole bei Temperaturen zwischen -20° und 200°C und einem Wasserstoffdruck von 1 bis 50 bar durchgeführt wird.

8. Verfahren nach einem der vorhergehenden Ansprüche, **dadurch gekennzeichnet, daß** die Hydrierung der Phenole in Gegenwart aromatischer Lösungsmittel und eines mit Alkali- oder Erdalkalicarbonat oder -hydroxid dotierten Palladiumkatalysators stattfindet.

9. Verfahren nach einem der vorhergehenden Ansprüche zur Herstellung der Verbindungen der Formeln I1 bis I19: 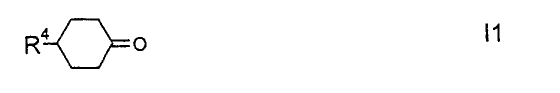 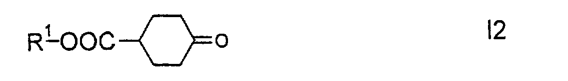 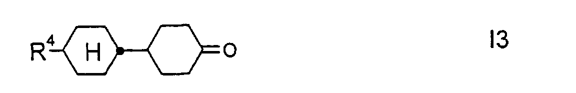 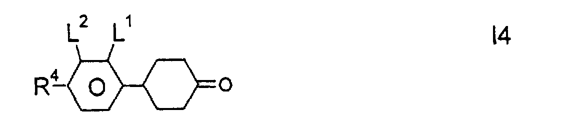 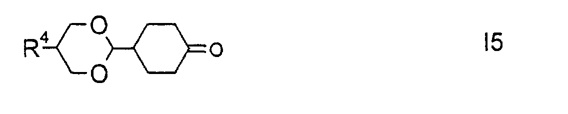 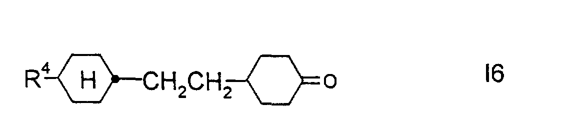 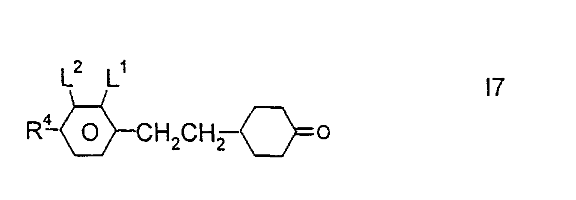 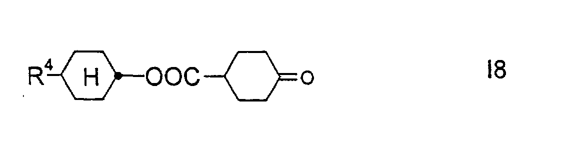 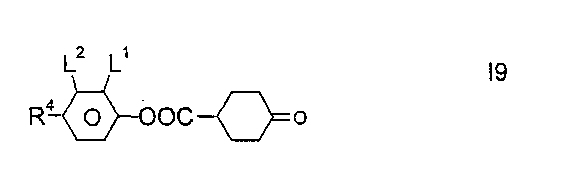 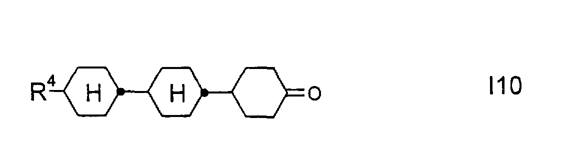 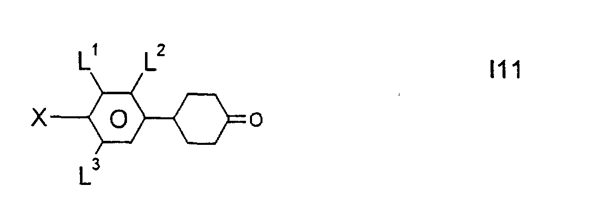 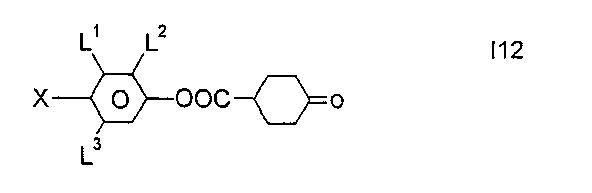 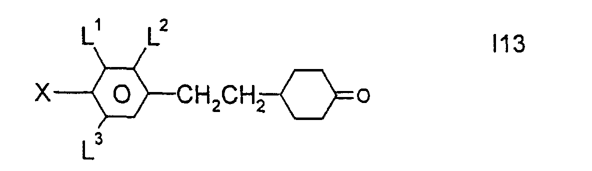 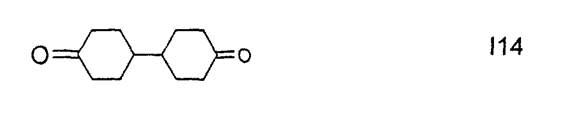 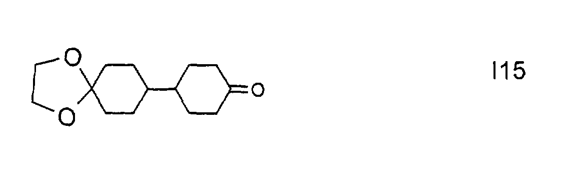 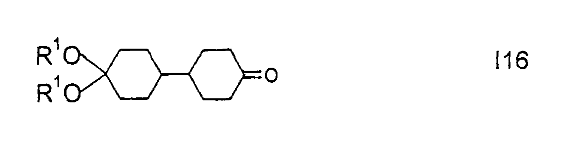 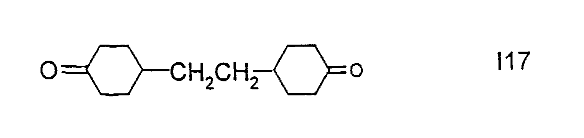 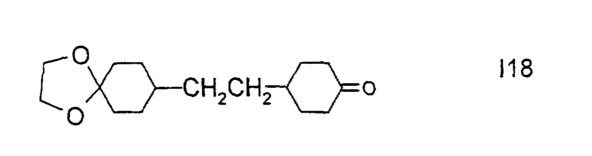 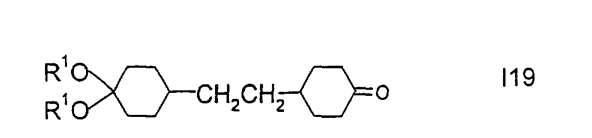 worin
R¹ die oben angegebene Bedeutung aufweist,
R⁴ -N(R¹)₂, -CON(R¹)₂, einen geradkettigen Alkylrest mit 1 bis 12 C-Atomen, wobei in diesem Rest auch eine oder mehrere CH₂-Gruppen durch-O- oder -CO- so ersetzt sein können, daß O-Atome nicht direkt miteinander verknüpft sind, bedeutet,
L¹, L², L³ unabhängig voneinander H oder F
und
X F, -CF₃, -OCF₃, -OCF₂CF₃ oder -OCHFCF₃
bedeutet.

## Claims

1. Process for the preparation of cyclohexanones from the corresponding phenols by partial hydrogenation, **characterised in that** the reaction mixture obtained by the hydrogenation is treated, before the isolation of the cyclohexanone, with chlorosulfonic acid or chlorosulfonic acid/ether adducts, amidosulfonic acid, sulfur trioxide in undiluted or nitrogen- or air-diluted form or sulfur trioxide/ether adducts in the presence of nitrogen bases.

2. Process according to Claim 1 for the preparation of cyclohexanones of the formula I 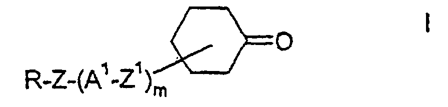 in which
R is one of the following chiral or achiral radicals: H, F, -CF₃, -OCF₃, -OCF₂CF₃ or -OCHFCF₃, -N(R¹)₂, -COOR¹, -CON(R¹)₂, -CHO, a straight-chain or branched alkyl radical having from 1 to 15 carbon atoms which is unsubstituted, monosubstituted by -CF₃ or at least monosubstituted by fluorine, where one or more non-adjacent CH₂ groups in these radicals may also, in each case independently of one another, be replaced by -S-, -O-, -CO-, -CO-O-, -O-CO-, -O-CO-O- or 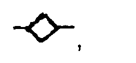 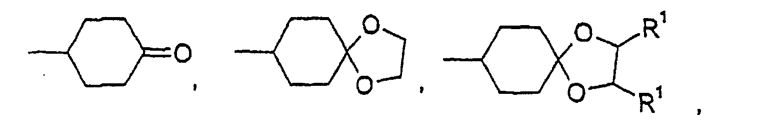 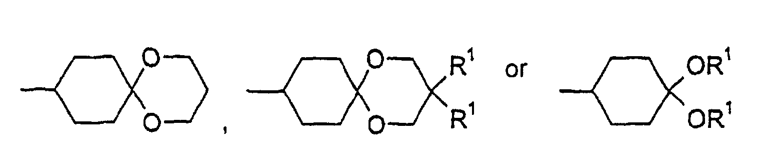
R¹ is an alkyl radical having from 1 to 12 carbon atoms,
A¹ is a
(a) cyclohexane-1,4-diyl radical, in which, in addition, one or more non-adjacent CH₂ groups may be replaced by -O-and/or -S-,
(b) 1,4-phenylene radical, in which, in addition, one or two CH groups may be replaced by N,
(c) radical from the group consisting of 2,6-dioxaborane-1,4-diyl, 1,4-bicyclo[2.2.2]octylene, piperidine-1,4-diyl, naphthalene-2,6-diyl, decahydronaphthalene-2,6-diyl and 1,2,3,4-tetrahydronapthalene-2,6-diyl,
where the radicals (a) and (b) may be substituted by one or more fluorine atoms,
Z and Z¹, independently of one another, are -CH₂O-, -OCH₂-, -CO-O-, -O-CO-, -CH₂S-, -SCH₂-, -CH₂CH₂- or a single bond,
m is 0, 1 or 2,
by partial hydrogenation of the corresponding phenols of the formula II 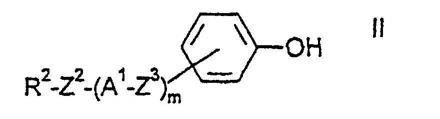 in which
R² adopts the meaning of R and in addition is also 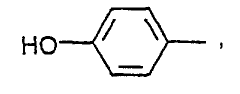
A¹ and m are as defined for the formula I, and
Z² and Z³, in addition to the meaning given under Z and Z¹, are alternatively -CH=CH- or -C≡C-.

3. Process according to one of Claims 1 and 2, **characterised in that** the reaction mixture obtained by the hydrogenation is treated with chlorosulfonic acid or chlorosulfonic acid/ether adducts, amidosulfonic acid, sulfur trioxide in undiluted or nitrogen- or air-diluted form or sulfur trioxide/ether adducts in an amount which corresponds to the molar fractions of the cyclohexanol and phenol.

4. Process according to one of Claims 1, 2 and 3, **characterised in that** the reaction mixture obtained by the hydrogenation is reacted with chlorosulfonic acid or chlorosulfonic acid/ether adducts, amidosulfonic acid, sulfur trioxide in undiluted or nitrogen- or air-diluted form or sulfur trioxide/ether adducts at temperatures of from -20°C to the boiling point of the respective least volatile component of the reaction mixture,.

5. Process according to one of Claims 1, 2, 3 and 4, **characterised in that** the reaction mixture obtained by the hydrogenation is treated with sulfur trioxide/pyridine adduct.

6. Process according to one of the preceding claims, **characterised in that** the reaction mixture obtained by the hydrogenation and treated with chlorosulfonic acid or chlorosulfonic acid/ether adducts, amidosulfonic acid, sulfur trioxide in undiluted or nitrogen- or air-diluted form or sulfur trioxide/ether adducts in the presence of nitrogen bases is subjected to filtration.

7. Process according to one of the preceding claims, **characterised in that** the hydrogenation of the phenols is carried out at temperatures between -20° and 200°C and a hydrogen pressure of from 1 to 50 bar.

8. Process according to one of the preceding claims, **characterised in that** the hydrogenation of the phenols is carried out in the presence of aromatic solvents and in the presence of a palladium catalyst doped with alkali or alkaline earth metal carbonate or hydroxide.

9. Process according to one of the preceding claims for the preparation of the compounds of the formulae I1 to I19: 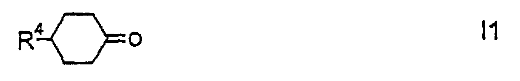 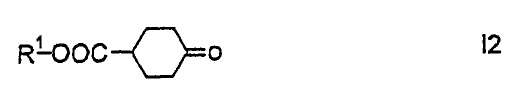 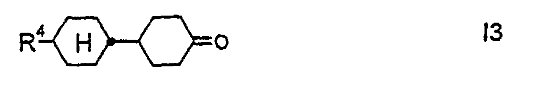 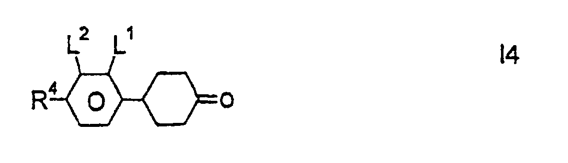 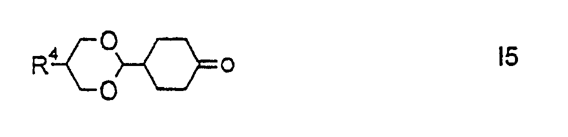 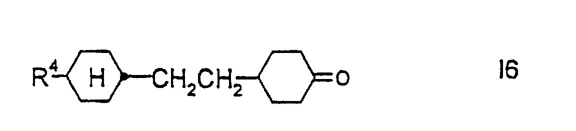 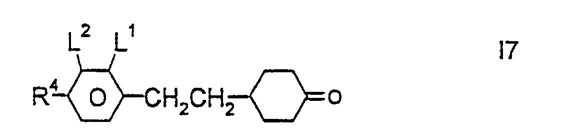 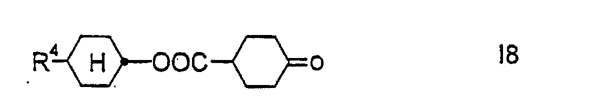 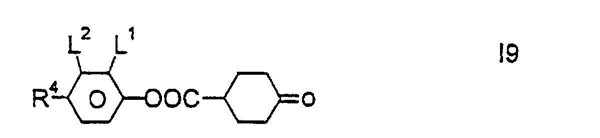 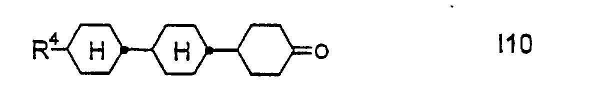 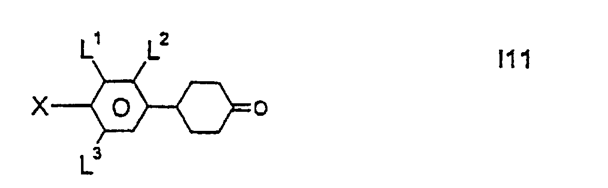 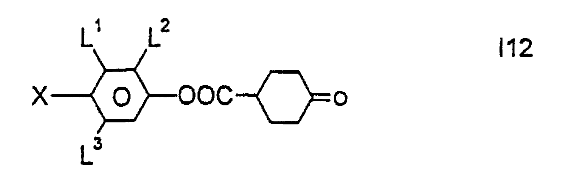 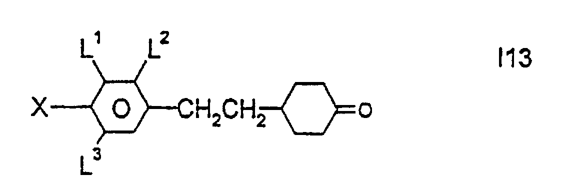 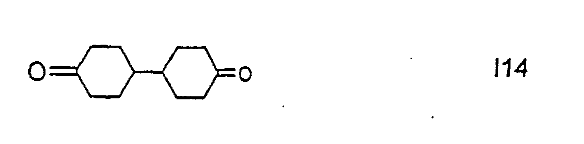 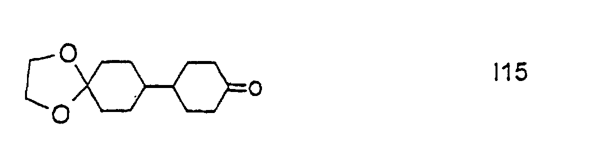 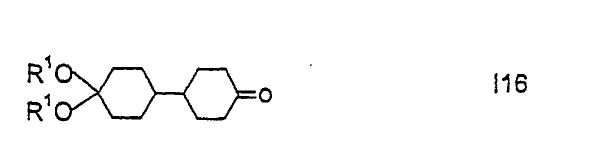 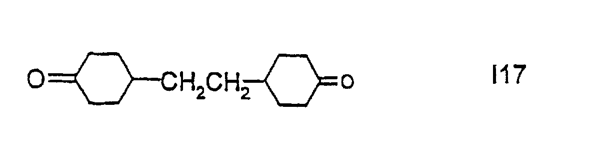 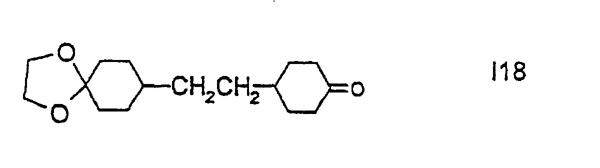 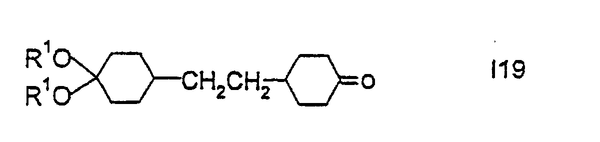 in which
R¹ is as defined above,
R⁴ is -N(R¹)₂, -CON(R¹)₂, a straight-chain alkyl radical having from 1 to 12 carbon atoms, where one or more CH₂ groups in this radical may also be replaced by -O- or -CO- in such a way that O atoms are not linked directly to one another,
L¹, L² and L³, independently of one another, are H or F,
and
X is F, -CF₃, -OCF₃, -OCF₂CF₃ or -OCHFCF₃.

## Revendications

1. Procédé pour la préparation de cyclohexanones à partir des phénols correspondants, par hydrogénation partielle, **caractérisé en ce qu'**on traite le mélange réactionnel obtenu par l'hydrogénation, avant l'isolement de la cyclohexanone, par de l'acide chlorosulfonique ou des adduits d'éther et d'acide chlorosulfonique, de l'acide amidosulfonique, de l'anhydride sulfurique sous forme non diluée ou diluée avec de l'azote ou de l'air, ou des adduits d'anhydride sulfurique et d'éther, en présence de bases azotées.

2. Procédé selon la revendication 1, pour la préparation de cyclohexanones de formule I 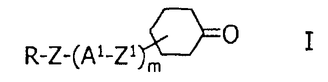 dans laquelle
R représente l'un des radicaux chiraux ou non chiraux suivants : H, F, -CF₃, -OCF₃, -OCF₂CF₃ ou -OCHFCF₃, -N(R¹)₂, -COOR¹, -CON(R¹)₂, -CHO, un radical alkyle à chaîne droite ou ramifié, ayant de 1 à 15 atomes de carbone, non substitué ou substitué une fois par -CF₃ ou au moins une fois par le fluor, dans ces radicaux un ou plusieurs groupes CH₂ non contigus pouvant également être remplacés chacun, indépendamment les uns des autres, par -S-, -O-, -CO-, -CO-O-, -O-CO-, -O-CO-O- ou 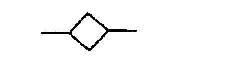 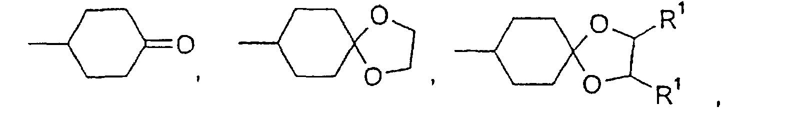 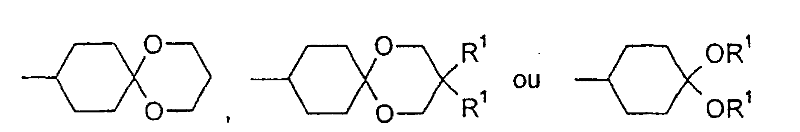
R¹ représente un radical alkyle ayant de 1 à 12 atomes de carbone,
A¹ représente
(a) un radical cyclohexane-1,4-diyle dans lequel un ou plusieurs groupes CH₂ non contigus pouvant également être remplacés par -O- et/ou -S-,
(b) un radical 1,4-phénylène dans lequel un ou deux groupes CH peuvent également être remplacés par N ;
(c) un radical choisi dans l'ensemble constitué par les groupes 2,6-dioxoborane-1,4-diyle, 1,4-bicyclo(2,2,2)-octylène, pipéridine-1,4-diyle, naphtalène-2,6-diyle, décahydronaphtalène-2,6-diyle et 1,2,3,4-tétrahydronaphtalène-2,6-diyle,
les radicaux (a) et (b) pouvant être substitués par un ou plusieurs atomes de fluor,
Z, Z¹ représentent, indépendamment l'un de l'autre, -CH₂O-, -OCH₂-, -CO-O-, -O-CO-, -CH₂S-, -SCH₂-, -CH₂CH₂- ou une liaison simple,
m est 0, 1 ou 2,
par hydrogénation partielle des phénols correspondants de formule II 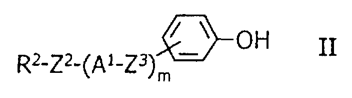 dans laquelle
R² adopte la signification de R et en outre représente également 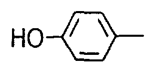
A¹, m ont les significations indiquées pour la formule I
et
Z², Z³, en plus de la signification indiquée pour Z et Z¹, représentent également -CH=CH- ou -C≡C-.

3. Procédé selon la revendication 1 ou 2, **caractérisé en ce que** le mélange réactionnel obtenu par l'hydrogénation est traité par une quantité d'acide chlorosulfonique ou d'adduits et d'acide chlorosulfonique et d'éther, d'acide amidosulfonique, d'anhydride sulfurique sous forme non diluée ou diluée avec de l'azote ou de l'air, ou d'adduits d'anhydride sulfurique et d'éther, qui correspond aux quantités molaires du cyclohexanol et du phénol.

4. Procédé selon l'une quelconque des revendications 1, 2 et 3, **caractérisé en ce qu'**on fait réagir le mélange réactionnel, obtenu par l'hydrogénation, avec de l'acide chlorosulfonique ou des adduits d'éther et d'acide chlorosulfonique, de l'acide amidosulfonique, de l'anhydride sulfurique sous forme non diluée ou diluée avec de l'azote ou de l'air, ou des adduits d'anhydride sulfurique et d'éther, à des températures de -20°C au point d'ébullition du composant le moins volatil du mélange réactionnel.

5. Procédé selon l'une quelconque des revendications 1, 2, 3 et 4, **caractérisé en ce que** le mélange réactionnel obtenu par l'hydrogénation est traité par un adduit d'anhydride sulfurique et de pyridine.

6. Procédé selon l'une quelconque des revendications précédentes, **caractérisé en ce que** le mélange réactionnel obtenu par l'hydrogénation et traité par de l'acide chlorosulfonique ou des adduits d'éther et d'acide chlorosulfonique, de l'acide amidosulfonique, de l'anhydride sulfurique sous forme non diluée ou diluée avec de l'azote ou de l'air, ou des adduits d'anhydride sulfurique et d'éther, en présence de bases azotées, est soumis à une filtration.

7. Procédé selon l'une quelconque des revendications précédentes, **caractérisé en ce que** l'hydrogénation des phénols est effectuée à des températures comprises entre -20°C et 200°C et sous une pression d'hydrogène de 1 à 50 bars.

8. Procédé selon l'une quelconque des revendications précédentes, **caractérisé en ce que** l'hydrogénation des phénols a lieu en présence de solvants aromatiques et d'un catalyseur à base de palladium dopé avec un hydroxyde ou carbonate de métal alcalin ou alcalino-terreux.

9. Procédé selon l'une quelconque des revendications précédentes, pour la préparation des composés de formules I1 à I19 : 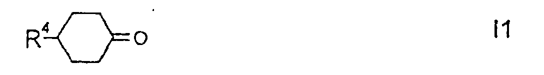 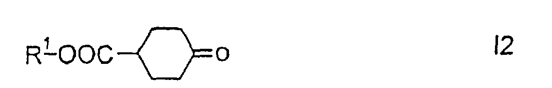 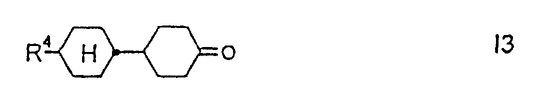 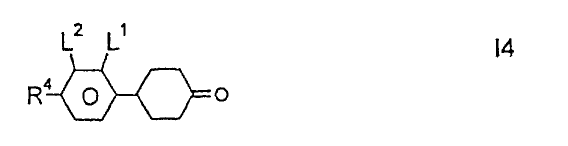 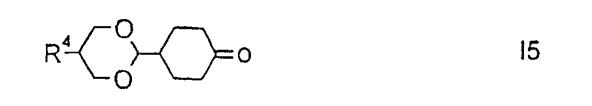 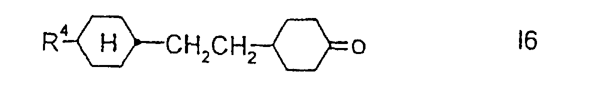 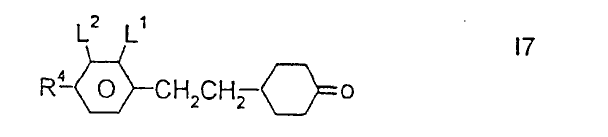 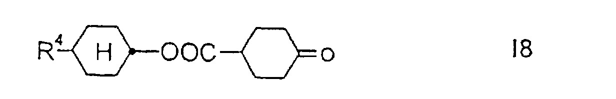 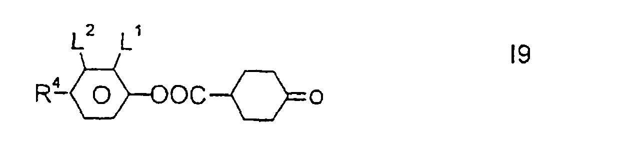 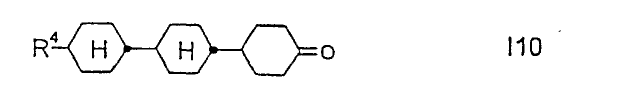 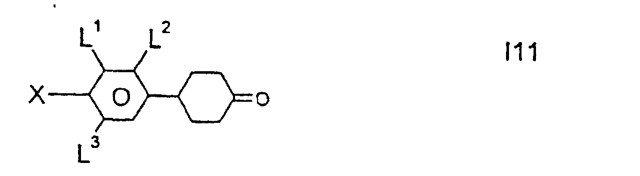 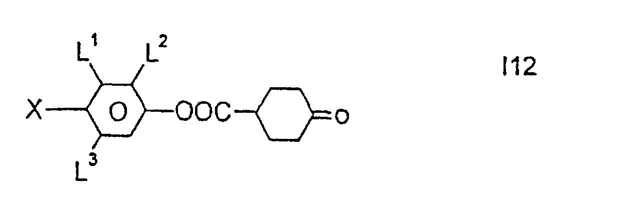 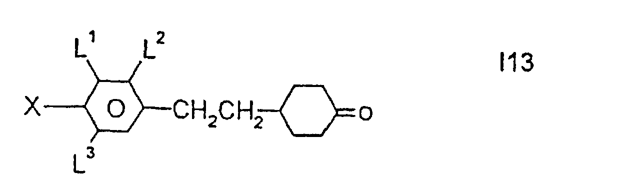 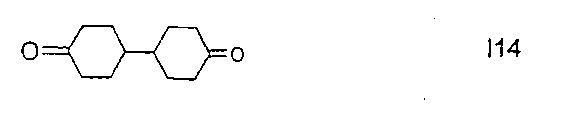 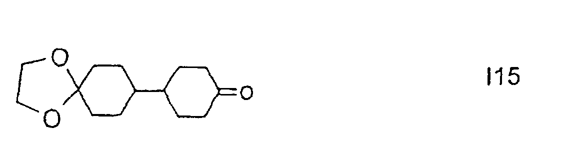 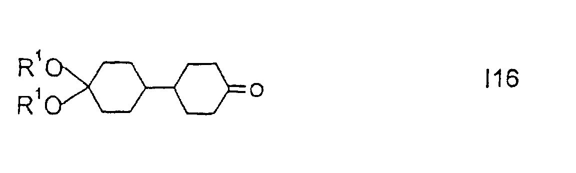 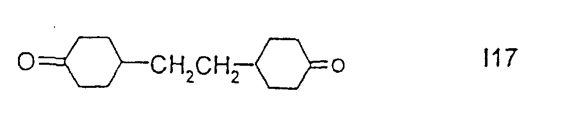 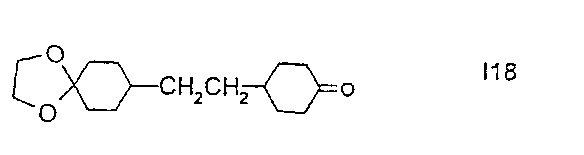 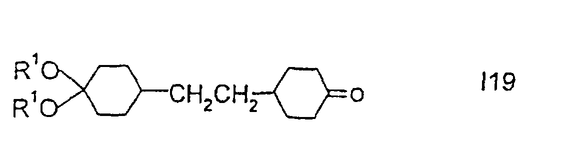 dans lesquelles
R¹ a la signification donnée plus haut,
R⁴ représente -N(R¹)₂, -CON(R¹)₂, un radical alkyle à chaîne droite ayant de 1 à 12 atomes de carbone, dans ce radical un ou plusieurs groupes CH₂ pouvant également être remplacés par -O- ou -CO-, de manière que des atomes d'oxygène ne soient pas reliés directement entre eux,
L¹, L², L³ représentent, indépendamment les uns des autres, H ou F,
et
X représente F, -CF₃, -OCF₃, -OCF₂CF₃ ou -OCHFCF₃.
